(19) Europäisches Patentamt
European Patent Office
Office européen des brevets

(11) Publication number: **0 191 884**
**B1**

(12) # EUROPEAN PATENT SPECIFICATION

(45) Date of publication of patent specification: **04.05.88**

(51) Int. Cl.$^4$: **C 07 C 49/04**, C 07 C 45/72, C 07 C 45/73

(21) Application number: **85101943.0**

(22) Date of filing: **22.02.85**

(54) **Process for the single-stage production of higher aliphatic ketones.**

(43) Date of publication of application:
**27.08.86 Bulletin 86/35**

(45) Publication of the grant of the patent:
**04.05.88 Bulletin 88/18**

(84) Designated Contracting States:
**AT BE CH DE FR GB LI LU NL SE**

(58) References cited:
**DE-A-2 022 365**
**US-A-2 262 817**

**CHEMICAL ABSTRACTS, vol. 77, 1972, page 360, no. 164039k, Columbus, Ohio, US**

(73) Proprietor: **CONSIGLIO NAZIONALE DELLE RICERCHE**
**Piazzale Aldo Moro, 7**
**I-00198 Roma (IT)**

(72) Inventor: **Fragale, Carlo**
**Via Onissanti, 38**
**I-70010 Capurso Bari (IT)**
Inventor: **Gargano, Michele**
**Via Papalia no. 7**
**I-70126 Bari (IT)**
Inventor: **Rossi, Michele**
**Via de Laurentis 14**
**Bari (IT)**

(74) Representative: **Gervasi, Gemma, Dr. et al**
**Studio Brevetti e Marchi NOTARBARTOLO & GERVASI 33, Viale Bianca Maria**
**I-20122 Milano (IT)**

**EP 0 191 884 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new process for the production of higher aliphatic ketones from ketones of lower molecular weight.

Processes for the production, for example, of methylisobutylketone from acetone are well known. This production is effected in three stages in the conventional process:

— the first stage consists of the aldol condensation of the acetone (I), catalysed by a base, to produce diacetone alcohol (II):

$$2CH_3{-}CO{-}CH_3 \overset{base}{\rightleftarrows} CH_3{-}\underset{\displaystyle CH_3}{\underset{|}{C}}OH{-}CH_2{-}CO{-}CH_3$$

I II

— the second stage consists of the removal of $H_2O$, catalysed in the liquid phase by $H_2SO_4$ or $H_3PO_4$, at a temperature of about 100°C to obtain mesityl oxide (III):

$$CH_3{-}\underset{\displaystyle CH_3}{\underset{|}{C}}OH{-}CH_2{-}CO{-}CH_3 \overset{H_2SO_4}{\rightleftarrows} CH_3{-}\underset{\displaystyle CH_3}{\underset{|}{C}}{=}CH{-}CO{-}CH_3+H_2O$$

III

— the third stage consists of the hydrogenation of the mesityl oxide at a temperature of 150°—200°C and a pressure of 3—10 bars in the presence of copper or nickel catalysts, to produce methylisobutylketone (IV):

$$CH_3{-}\underset{\displaystyle CH_3}{\underset{|}{C}}{=}CH{-}CO{-}CH_3 \overset{H_2}{\rightleftarrows} CH_3{-}\underset{\displaystyle CH_3}{\underset{|}{C}}H{-}CH_2{-}CO{-}CH_3$$

IV

In order to simplify this process, various methods have been proposed which attempt to reduce the three stages to a single stage by operating in the presence of suitably prepared catalysts (for example French patent 2,056,450; Japanese patents 74/06,290 and 72/20112; British patent 1,269,891; French patent 2,064,778; DE—OS 2 022 365).

The catalytic systems used in these single-stage processes generally consist of one or more transition metals (Ru, Rh, Ir, Ni, Pd, Pt, Cu, Cr etc.) dispersed on matrices able to catalyse the condensation of acetone to α,β unsaturated ketones (CaO, MgO, $Al_2O_3$, $SiO_2$, $TiO_2$, ZnO etc.).

The reduction is conducted using gaseous hydrogen at a pressure of between 1 and 50 bars, and operating at a temperature of between 100° and 350°C.

The results of the reaction in terms of selectivity vary considerably with the degree of acetone conversion, the reaction conditions and the catalytic system used. The best results have been obtained with a Pd-Zr-P catalytic system.

We have now discovered a new process for the production of higher aliphatic ketones from ketones of lower molecular weight, operating in a single stage.

The process according to the present invention is characterised in that the starting ketone is reacted in the presence of CO over a catalyst in the form of copper supported on a metal oxide.

The process according to the present invention is further characterised in that the catalyst is prepared by precipitating, by hydrolysis, a cuprammonium complex on an oxide such as $Al_2O_3$, $SiO_2$, $TiO_2$, $ZrO_2$, MgO, ZnO, $Cr_2O_3$ etc., followed by calcining in air and reduction in a hydrogen stream.

A further characteristic of the process according to the present invention is that the reaction can be conducted either in the liquid phase or in the gaseous phase.

A possible theoretical interpretation of the phenomena which occur in the process of the present invention can be represented by the following scheme, which relates to the preparation of methylisobutylketone starting from acetone.

1) $$2CH_3{-}CO{-}CH_3 \rightleftarrows CH_3{-}\underset{\displaystyle CH_3}{\underset{|}{C}}OH{-}CH_2{-}CO{-}CH_2$$

2) $$CH_3{-}COH{-}CH_2{-}CO{-}CH_3 \rightleftarrows CH_3{-}\underset{\displaystyle CH_3}{\underset{|}{C}}{=}CH{-}CO{-}CH_3+H_2O$$

3) $$H_2O+CO \rightleftarrows H_2+CO_2$$

4) 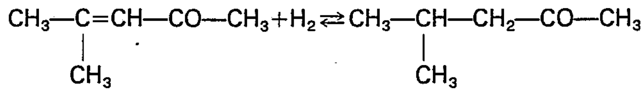

Overall, the process can be represented by the following reaction:

5) 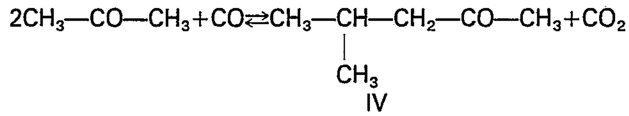

IV

The methylisobutylketone (IV) can further react to give ketones of higher molecular weight, for example it can react in accordance with the following reactions:

6) 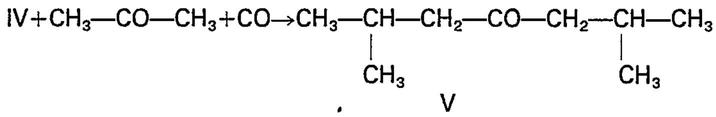

V

7) 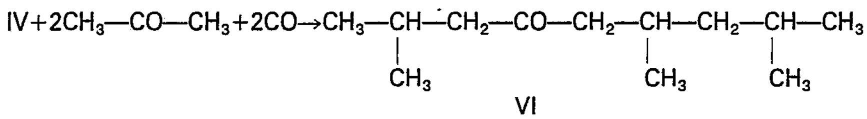

VI

In this manner, diisobutylketone (V) and 2,4,8-trimethylnonan-6-one (VI) are obtained.

According to the aforesaid scheme, the hydrogen necessary for reducing the α,β unsaturated ketones is produced in-situ by the water gas conversion reaction catalysed by the copper dispersed on the catalyst surface (reaction 3).

An advantage of the process according to the present invention is therefore the fact that the water produced in condensing the acetone to mesityl oxide (reactions 1 and 2) is completely consumed in producing the hydrogen necessary for reducing the unsaturated ketone (reaction 4) and this tends to further increase the degree of advancement of reaction 2.

The catalyst for use in the process according to the present invention is preferably prepared by the following method, although other catalyst preparation methods can be used within the scope of the invention.

An aqueous solution of a cuprammonium complex is prepared starting from $Cu(NO_3)_2 \cdot 3H_2O$, $CuSO_4 \cdot 10/H_2O$, $Cu(ClO_4)_2 \cdot 6H_2O$ or other copper salts, the solution having a Cu content of between 2 and 20 g/l and preferably between 12 and 15 g/l, and being stabilised with a slight excess of ammonia.

$Al_2O_3$ is added to this solution in a quantity such as to obtain a weight ratio of Cu to $Al_2O_3$ of between 0.015 and 0.15, and preferably between 0.085 and 0.11, said $Al_2O_3$ having the following characteristics: crystalline structure type γ, particle size distribution between 50 and 150 μm (100—290 mesh ASTM), specific surface preferably between 150 and 200 $m^2/g$.

The suspension is diluted with distilled water until the copper concentration lines between 0.2 and 6 g/l, and preferably between 0.9 and 1.2 g/l.

During the dilution, the suspension is kept under agitation (500 r.p.m.) at a temperature preferably of between 10° and 30°C.

By virtue of the dilution, the cuprammonium complex hydrolyses, and the hydrolysis product is deposited in a finely dispersed form on the surface of the $Al_2O_3$ particles.

The solid is then filtered, after which it is washed three times with between 30 and 50 ml/g of distilled water, the product then being calcined in air at a temperature of between 250° and 400°C for a time of between 1 and 10 hours, and preferably for a time of between 3 and 4 hours. The calcined product is then reduced in a hydrogen stream by forming the catalyst into a fixed bed and passing the hydrogen over it at a flow rate of between 1 and 100 ml/min. The reduction temperature is kept constant at between 150° and 350°C.

The reaction time is between 5 and 90 minutes, and preferably between 10 and 20 minutes.

The catalyst thus obtained consists of Cu supported on $Al_2O_3$. The Cu content of the catalyst is between 5 and 10%, the particle size distribution is between 50 and 100 μm (100—290 mesh ASTM), and the specific surface is between 200 and 250 $m^2/g$.

Other oxides can be used instead of the $Al_2O_3$ for the catalyst support, such as $SiO_2$, $TiO_2$, $ZrO_2$, MgO or ZnO.

The reaction for producing higher ketones from acetone in the liquid phase is conducted in the following manner.

The acetone and catalyst are fed into a metal reactor in an acetone/Cu molar ratio of between 50 and

500 and preferably between 90 and 150. Carbon monoxide is then fed in until a pressure of between 0.5 and 80 bars and preferably between 15 and 40 bars is reached. The reactor is heated to a temperature of between 120° and 220°C and preferably between 140° and 200°C. The reaction is conducted for a time of between 0.25 and 15 hours and preferably between 0.5 and 4 hours.

If the required product is methylisobutylketone, it is convenient to operate at the lower limits of the reaction pressure, temperature and time ranges, compatible with a reasonable level of acetone conversion, for example around 50%. At the higher limits of these ranges substantial quantities of diisobutylketone form at the expense of the methylisobutylketone.

The reaction for producing higher ketones in the liquid phase from ketones other than acetone, for example from butan-2-one, from methylisobutylketone etc., is conducted in the same manner as described for acetone, but with the difference that the reaction time has to be increased, preferably up to 14 hours.

When operating with butan-2-one, with a conversion exceeding 65%, 3-methylheptan-5-one can be obtained with a selectivity exceeding 90%. When operating with methylisobutylketone, with a conversion exceeding 85%, 2-4-8-thiomethylnonan-6-one can be obtained with a selectivity exceeding 90%.

The process according to the present invention can also be conducted in the gaseous phase. In this case, the catalyst is fed into a tubular reactor which is heated to a temperature of between 120° and 220°C and preferably between 140° and 180°C. A gaseous stream of carbon monoxide and ketone, preferably in a molar ratio close to 2/1, is passed through the reactor at a pressure slightly greater than atmospheric pressure.

The results obtained by operating in the gaseous phase are analogous to those obtained by operating in the liquid phase.

The following examples are given in order to illustrate the operational method and characteristics of the process according to the present invention.

Example 1

20 g of γ $Al_2O_3$ of particle size distribution between 50 and 150 μm (100—290 mesh ASTM) and specific surface 175 $m^2/g$ were added to a solution containing 8 g of cuprammonium complex prepared from $Cu(NO_3)_2 3H_2O$ in 150 ml of water, stabilised by adding 3 ml of concentrated ammonia.

The mixture thus obtained was diluted with 2 liters of distilled water at a temperature of 25°C, maintaining energetic agitation (500 r.p.m.) during the dilution, so as to cause hydrolysis of the cuprammonium complex and deposition of the hydrolysis product in finely dispersed form on the surface of the $Al_2O_3$ granules.

The solid product was separated by filtration, washed with three portions of distilled water each of 1 litre volume, calcined in air at 350°C for 180 minutes, and finally reduced in a hydrogen stream at 270°C for 15 minutes by forming it into a layer having a thickness of 1 cm and passing hydrogen over it at a throughput of 20 ml/min.

A catalyst was obtained constituted by Cu supported on $Al_2O_3$, containing 4.5% of Cu and in the form of particles of size distribution 50—150 μm (100—290 mesh ASTM), and with a specific surface of 260 $m^2/g$.

Example 2

1 g of catalyst prepared as described in Example 1 and 5 ml of acetone (acetone/Cu molar ratio 96) were placed in a 100 ml AISI 316 stainless steel reactor provided with a cock, pressure gauge and mechanical agitator.

Carbon monoxide was fed through the reactor cock until a pressure of 40 bars was reached in the reactor. The carbon monoxide used had the following analytical characteristics: $\%H_2=1.0$, $\%CO=99.0$.

The reaction temperature was rapidly raised to 180°C by means of a temperature-controlled oven, and as soon as heating started the reactor was put under vigorous agitation.

The reaction time in the test of this example was 0.5 hours. At the end of the test, the reactor was cooled rapidly by a stream of water, and after evacuating the gaseous atmosphere the reactor was opened in order to separate the catalyst from the reaction product by filtration.

When analysed by gas chromatography, the reaction product was found to consist of a high percentage of methylisobutylketone, and smaller percentages of diisobutylketone and other compounds, as shown in Table 1 accompanying Examples 3 to 5.

Examples 3 to 5

Examples 3 to 5 were conducted in the same manner as Example 2, but with the difference that the reaction times were 1, 2 and 4 hours respectively.

The products obtained were analysed by gas chromatography, and the results are shown in the following Table 1:

TABLE 1

| Ex. No. | Reaction time (hours) | Acetone convers. % | Products % by weight | | | | | | | |
|---|---|---|---|---|---|---|---|---|---|---|
| | | | IPA | MOX | MIBK | MIBC | DIBK | DIBC | C12 | Others |
| 2 | 0.5 | 36.7 | 7.8 | 7.9 | 75.9 | — | 8.4 | — | — | 0.2 |
| 3 | 1 | 77.3 | 2.1 | 0.4 | 74.6 | 2.3 | 18.8 | 0.4 | 1.5 | 0.7 |
| 4 | 2 | 88.8 | 1.1 | 0.1 | 69.1 | 0.9 | 25.9 | 0.2 | 2.7 | 1.5 |
| 5 | 4 | 96.0 | 0.8 | — | 59.8 | 4.0 | 29.7 | 1.1 | 4.6 | 2.8 |

in which the symbols have the following meanings: IPA=isopropanol; MOX=mesityl oxide; MIBK=methylisobutylketone; MIBC=methylisobutylcarbinol; DIBK=diisobutylketone; DIBC=diisobutyl-carbinol.

Examples 6 to 8

These examples were conducted in the same operating manner as Example 2, but varying the reaction parameters, namely carbon monoxide pressure, reaction temperature and reaction time, as shown in the following Table 2:

TABLE 2

| Ex. No. | CO pressure (bars) | Temperature (°C) | Reaction time (hours) |
|---|---|---|---|
| 6 | 40 | 140 | 4 |
| 7 | 40 | 220 | 1 |
| 8 | 15 | 180 | 4 |

The products obtained were analysed by gas chromatography, and the results are shown in the following Table 3:

TABLE 3

| Ex. No. | Acetone conversion (%) | Products % by weight | | | | | | | |
|---|---|---|---|---|---|---|---|---|---|
| | | IPA | MOX | MIBK | MIBC | DIBK | DIBC | C12 | Others |
| 6 | 52.4 | 6.2 | 5.4 | 83.1 | — | 5.3 | — | — | 0.5 |
| 7 | 92.6 | 1.6 | 0.1 | 58.6 | 4.6 | 29.0 | 1.5 | 4.9 | 2.0 |
| 8 | 63.9 | 0.7 | 6.7 | 78.5 | — | 13.6 | — | 0.5 | 3.4 |

in which the symbols have the meanings associated with Table 1.

Example 9 (comparison)

This example was conducted in the same operating manner as Example 2, but with a reaction time of 4 hours and using $Al_2O_3$ as catalyst, without the Cu.

Gas chromatography analysis of the reaction products showed an acetone conversion of 17.6%, the product consisting of 86.5% mesityl oxide and 15.5% of unidentified components.

Example 10 (comparison)

The procedure of Example 2 was followed, but with a reaction time of 4 hours and using nitrogen instead of carbon monoxide. The acetone conversion was 16.2%, and the product consisted of 89% mesityl oxide and 11% of unidentified compounds.

Example 11

In this example, in contrast to the preceding examples, the reaction was conducted in the gaseous phase in the following manner. 10 g of catalyst prepared as described in Example 1 were placed in a horizontal reactor constituted by a glass tube of 1 cm inner diameter. The catalyst was disposed in a layer of height 10 mm.

The temperature was raised to 180°C by means of a tubular oven, and a gaseous stream of carbon monoxide and acetone in a molar ratio of 2/1 was passed through the reactor. The feed throughput was 0.3 millimoles of acetone per minute, and the pressure inside the reactor was maintained at 1.3 bars.

After an initial period of 3 hours, the reaction products were collected by means of a trap kept at a temperature of −15°C.

Gas chromatography analysis showed an acetone conversion of 97%, the product consisting of 51% methylisobutylketone, 34% diisobutylketone, 6% 2,4,8-trimethylnonan-6-one, 3.5% mesityl oxide, and 4.5% of unidentified products.

Example 12

This example was conducted in the manner of Example 2, but with a reaction time of 3 hours and using butan-2-one instead of acetone.

Gas chromatography analysis of the reaction mixture showed a butan-2-one conversion of 68.1%, the mixture composition being 94.3% 5-methylheptan-3-one, 3.6% butan-2-ol, and 2.1% of other unidentified compounds.

Example 13

This example was conducted in the manner of Example 2, but with a reaction time of 14 hours and using methylisobutylketone instead of acetone.

Gas chromatography analysis of the reaction mixture showed a methylisobutyl conversion of 86.1%, the reaction mixture consisting of 93% 2,4,8-trimethylnonan-6-one, 4.2% methylisobutylcarbinol, and 2.7% of other unidentified compounds.

**Claims**

1. A single stage process for the production of higher aliphatic ketones starting from ketones of lower molecular weight, characterized in that the ketones of lower molecular weight are reacted with carbon monoxide at temperature comprised between 120°C and 220°C at a carbon monoxide partial pressure between 0,5 and 80 bars and in presence of a catalyst consisting of copper supported on aluminium oxide and obtained by precipitation of copper compounds on the Al oxide support, through hydrolysing of an aqueous solution of cupro-ammonium complex, subsequent calcination of the copper precipitate and then reduction with hydrogen at temperature between 150° and 350°C.

2. A process as claimed in claim 1, wherein the system constituted by the Al oxide and copper precipitate is calcined in air at a temperature of between 250°C and 400°C for a time of between 1 and 10 hours and preferably between 3 and 4 hours.

3. A process as claimed in claim 1, wherein the system constituted by the Al oxide with the copper deposit originating from the calcining operation is reduced with hydrogen at a temperature of between 150°C and 350°C for a time of between 5 and 90 minutes and preferably between 10 and 20 minutes.

4. A process as claimed in claim 1, characterized in that the starting ketones are reacted with carbon monoxide in the liquid phase.

5. A process as claimed in claim 1, characterized in that the starting ketones are reacted with carbon monoxide in the gaseous phase.

6. A process as claimed in claim 5, wherein the catalyst is fed into a tubular reactor heated to a temperature of between 120°C and 220°C and preferably between 140°C and 180°C, while passing through the reactor a gaseous stream constituted by carbon monoxide and the starting ketone preferably in a molar ratio close to 2/1, at a pressure slightly exceeding atmospheric pressure.

**Patentansprüche**

1. Einstufenverfahren zur Herstellen von höheren aliphatischen Ketonen ausgehend von Ketonen mit niedrigerem Molgewicht, dadurch gekennzeichnet, daß die Ketone mit niedrigerem Molgewicht mit Kohlenmonoxid bei einer Temperatur von 120 bis 220°C bei einem Kohlenmonoxidpartialdruck von 0,5 bis 80 bar und in Anwesenheit eines Katalysators, bestehend aus Kupfer auf einem Aluminiumoxidträger, erhalten durch Ausfällung von Kupferverbindungen auf dem Aluminiumoxidträger durch Hydrolysieren einer wässerigen Lösung eines Kupferammoniumkomplexes, nachfolgende Kalzinierung des Kupferniederschlages und schließlich Reduktion mit Wasserstoff bei einer Temperatur von 150 bis 350°C, umgesetzt werden.

2. Verfahren, wie in Anspruch 1 beansprucht, worin das aus dem Aluminiumoxid und dem Kupferniederschlag bestehende System in Luft bei einer Temperatur von 250 bis 400°C während einer Zeit von 1 bis 10 Stunden, vorzugsweise 3 bis 4 Stunden, kalziniert wird.

3. Verfahren, wie in Anspruch 1 beansprucht, worin das aus dem Aluminiumoxid und dem Kupferniederschlag bestehende System, das vom Kalzinierungsvorgang stammt, mit Wasserstoff bei einer Temperatur von 150 bis 350°C während einer Zeit von 5 bis 90 Minuten, vorzugsweise 10 bis 20 Minuten, reduziert wird.

4. Verfahren, wie in Anspruch 1 beansprucht, dadurch gekennzeichnet, daß die Ausgangsketone mit Kohlenmonoxid in der flüssigen Phase umgesetzt werden.

5. Verfahren, wie in Anspruch 1 beansprucht, dadurch gekennzeichnet, daß die Ausgangsketone mit Kohlenmonoxid in der Gasphase umgesetzt werden.

6. Verfahren, wie in Anspruch 5 beansprucht, worin der Katalysator in einem rohrförmigen Reaktor eingebracht wird, der auf eine Temperatur von 120 bis 220°C, vorzugsweise 140 bis 180°C, erhitzt wird, während durch den Reaktor ein aus Kohlenmonoxid und dem Ausgangsketon, vorzugsweise in einem Molverhältnis nahe 2/1, bestehende Gasstrom bei einem Druck, der Atmosphärendruck leicht übersteigt, geleitet wird.

**Revendications**

1. Procédé en un seul stade pour la préparation de cétones aliphatiques supérieures en partant de cétones de masse moléculaire inférieure, caractérisé par le fait que les cétones de masse moléculaire inférieure sont mises à réagir avec de l'oxyde de carbone, à une température comprise entre 120 et 220°C, sous une pression partielle de l'oxyde de carbone comprise entre 0,5 et 80 bars et en présence d'un catalyseur constitué de cuivre sur support d'oxyde d'aluminium et obtenu par précipitation de composés du cuivre sur le support d'oxyde d'Al, par hydrolyse d'une solution aqueuse de complexe de cuprammonium, calcination ultérieure du précipité de cuivre puis réduction avec de l'hydrogène à une température comprise entre 150 et 350°C.

2. Procédé selon la revendication 1, dans lequel le système constitué par l'oxyde d'Al et le précipité de cuivre est calciné dans l'air à une température comprise entre 250 et 400°C pendant un temps compris entre 1 et 10 heures et de préférence entre 3 et 4 heures.

3. Procédé selon la revendication 1, dans lequel le système constitué par l'oxyde d'Al avec le dépôt de cuivre provenant de l'opération de calcination est réduit par de l'hydrogène à une température comprise entre 150°C et 350°C, pendant un temps compris entre 5 et 90 min. et de préférence entre 10 et 20 min.

4. Procédé selon la revendication 1, caractérisé par le fait que les cétones de départ sont mise à réagir avec de l'oxyde de carbon en phase liquide.

5. Procédé selon la revendication 1, caractérisé par le fait que les cétones de départ sont mises à réagir avec de l'oxyde de carbone en phase gazeuse.

6. Procédé selon la revendication 5, dans lequel le catalyseur est introduit dans un réacteur tubulaire chauffé à une température comprise entre 120°C et 220°C et de préférence entre 140°C et 180°C, tout en faisant passer à travers le réacteur un courant gazeux constitué d'oxyde de carbone et de la cétone de départ, de préférence dans un rapport molaire proche de 2/1 sous une pression dépassant légèrement la pression atmosphérique.